# EUROPÄISCHE PATENTSCHRIFT

(11) **EP 4 307 946 B1**
(45) Veröffentlichungstag und Bekanntmachung des Hinweises auf die Patenterteilung: **12.11.2025**
(21) Anmeldenummer: 22715067.9
(22) Anmeldetag: 16.03.2022
(51) Int. Cl.: A24F 40/44, A24F 40/48, A61M 11/04, A24F 40/42, A24F 40/485, A24F 40/51, A61M 15/06

(54) **INHALATOR**
INHALER
INHALATEUR

(30) Priorität: 16.03.2021 DE 102021202546
(43) Veröffentlichungstag der Anmeldung: 24.01.2024
(73) Patentinhaber: Alveon GmbH, 8807 Freienbach (CH)
(72) Erfinder: GEILEN, André, 8807 Freienbach (CH); RIESS, Ralf Martin, 8807 Freienbach (CH); GEISS, Simon, 8807 Freienbach (CH)
(74) Vertreter: BRP Renaud & Partner mbB Rechtsanwälte Patentanwälte Steuerberater
(86) Internationale Anmeldenummer: PCT/EP2022/056788
(87) Internationale Veröffentlichungsnummer: WO 2022/194915

(56) Entgegenhaltungen:
- EP-A1- 3 267 811
- US-A1- 2008 230 052
- US-A1- 2014 261 488
- US-A1- 2015 237 916
- US-A1- 2018 214 645
- US-A1- 2020 154 781
- US-A1- 2020 383 383
- US-A1- 2021 068 457
- US-B1- 6 234 167

## Beschreibung

Die vorliegende Erfindung betrifft einen Inhalator zum Inhalieren eines Aerosols, welcher einen Behälter zum Bevorraten einer zur Verdampfung vorgesehenen Flüssigkeit sowie eine Verdampfereinrichtung zum Verdampfen der Flüssigkeit und somit Erzeugen des Aerosols aufweist.

Mit einem Inhalator wird ein Aerosol zum Inhalieren erzeugt. Zu diesem Zweck wird in der Regel eine Flüssigkeit in einer Verdampfereinrichtung verdampft und somit ein Aerosol erzeugt und ausgegeben. Die Verdampfereinrichtung umfasst gewöhnlich eine Aufnahmestruktur zum Aufnehmen der zu verdampfenden Flüssigkeit sowie einen elektrischen Verdampfer zum Verdampfen der Flüssigkeit.

Aus der DE 10 2016 120 803 A1 ist ein Inhalator mit einer Verdampfereinrichtung bekannt. Die Verdampfereinrichtung weist einen Verdampfer aus einer dotierten und elektrisch leitfähigen Keramik auf. Die Keramik ist mit geregelten und eine vorgegebene Orientierung aufweisenden Mikrokanälen versehen, durch welche Flüssigkeit zwecks Verdampfens strömt. Die Keramik erzeugt bei elektrischer Versorgung Wärme, um die darin aufgenommene Flüssigkeit zu verdampfen. Die Verdampfereinrichtung weist ferner eine Durchflusssteuerungseinrichtung auf, welche den Durchfluss der Flüssigkeit durch die Mikrokanäle steuert.

Aus der WO 2004 022 242 A1 ist ein Inhalator bekannt, welcher eine Verdampfungseinrichtung mit einer beheizten Einzelkapillare aufweist. Der Inhalator weist ferner einen Behälter zum Bevorraten einer Flüssigkeit auf, welche zur Einzelkapillare strömen kann. Die Strömung der Flüssigkeit zur Einzelkapillare wird über ein Ventil geregelt.

Aus der US 20 2000 022 416 A1 ist ein Inhalator bekannt, der einen Behälter zum bevorraten einer Flüssigkeit aufweist. Im Behälter ist ein Kolben geführt, der beim Verstellen Flüssigkeit aus dem Behälter auf einen Verdampfer bringt. Der Verdampfer erzeugt im Betrieb Wärme, um die auf den Verdampfer aufgebrachte Flüssigkeit zu verdampfen.

Weitere Inhalatoren sind aus der US 2020/154781 A1 und der US 2020/383383 A1 bekannt.

Die vorliegende Erfindung beschäftigt sich mit der Aufgabe, für einen Inhalator der eingangs genannten Art sowie für eine Einheit für einen solchen Inhalator verbesserte oder zumindest andere Ausführungsformen anzugeben, welche sich insbesondere durch eine verbesserte Kontrolle der verdampften Flüssigkeit auszeichnen.

Diese Aufgabe wird erfindungsgemäß durch die Gegenstände der unabhängigen Ansprüche gelöst. Vorteilhafte Ausführungsformen sind Gegenstand der abhängigen Ansprüche.

Die vorliegende Erfindung beruht auf dem allgemeinen Gedanken, einen Inhalator mit einer Verdampfereinrichtung bereitzustellen, welche eine Aufnahmestruktur mit einem vorgegebenen Gesamtvolumen zur Aufnahme einer zu verdampfenden Flüssigkeit aufweist, wobei die Aufnahmestruktur mittels einer fluidischen Verbindung mit einem Behälter zum Bevorraten der Flüssigkeit mit Flüssigkeit befüllt und anschließend vom Behälter fluidisch getrennt wird, und wobei die in der Aufnahmestruktur aufgenommene Flüssigkeit im vom Behälter fluidisch getrennten Zustand der Aufnahmestrukturen zum Erzeugen des Aerosols verdampft wird. Das vorgegebene und somit bekannte Gesamtvolumen der Aufnahmestruktur führt dazu, dass beim Verdampfen der in der Aufnahmestruktur aufgenommenen Flüssigkeit die Menge oder zumindest maximal mögliche Menge der verdampften Flüssigkeit bekannt und somit genau kontrollierbar ist. Dies führt zu einer verbesserten Kontrolle der beim Verdampfen jeweils verdampften Menge der Flüssigkeit bekannt und somit genau kontrollierbar ist. Dies führt zu einer verbesserten Kontrolle der beim Verdampfen jeweils verdampften Menge der Flüssigkeit und somit zu einer verbesserten Kontrolle des zum Inhalieren ausgegebenen Aerosols. In der Summe wird also somit eine verbesserte Kontrolle über den Verdampfungsprozess und der verdampften Menge erreicht. Somit ist es insbesondere möglich, denn Inhalator zur medizinischen Anwendung einzusetzen.

Dem Erfindungsgedanken entsprechend weist der Inhalator den Behälter zum Bevorraten einer Flüssigkeit sowie die Verdampfereinrichtung auf. Die Verdampfereinrichtung umfasst die Aufnahmestruktur sowie einen elektrischen Verdampfer zum Verdampfen der Flüssigkeit und somit Erzeugen des Aerosols. Die Aufnahmestruktur weist ein vorgegebenes und somit bekanntes Gesamtvolumen zum Aufnahmen der Flüssigkeit auf. Der Inhalator ist zwischen einem Füllzustand und einem Verdampfungszustand verstellbar. Im Füllzustand sind die Aufnahmestruktur und der Behälter fluidisch miteinander verbunden, sodass im Behälter bevorratete Flüssigkeit in die Aufnahmestruktur gelangt. Demgegenüber sind im Verdampfungszustand die Aufnahmestruktur und der Behälter fluidisch voneinander getrennt, sodass ein Strömen von Flüssigkeit vom Behälter in die Aufnahmestruktur unterbunden ist. Dabei wird im Verdampfungszustand in der Aufnahmestruktur aufgenommene Flüssigkeit mittels des elektrischen Verdampfers zum Erzeugen des Aerosols verdampft. Zu diesem Zweck weist der Inhalator eine entsprechend ausgestaltete Steuereinrichtung auf. Die Steuereinrichtung ist folglich derart ausgestaltet, dass sie den Verdampfer im Verdampfungszustand elektrisch versorgt, sodass in der Aufnahmestruktur aufgenommene Flüssigkeit verdampft.

Der Verdampfer ist zweckmäßig derart ausgestaltet, dass er im Verdampfungszustand bei elektrischer Versorgung Wärme zum Verdampfen der in der Aufnahmestruktur aufgenommenen Flüssigkeit erzeugt. Insbesondere kann der Verdampfer als ein Heizwiderstand ausgebildet sein oder einen Heizwiderstand aufweisen.

Bevorzugt handelt es sich bei dem Inhalator um einen mobilen und händisch tragbaren Inhalator, der also mitgetragen werden kann. Der Inhalator kann bevorzugt von einem Nutzer händisch umgriffen und getragen werden. Entsprechend ist der Inhalator hinsichtlich seiner Abmessungen ausgebildet.

Bevorzugt weist der Inhalator eine Batterie, vorzugsweise eine wiederaufladbare Batterie, zur elektrischen Versorgung auf. Dabei ist die Steuereinrichtung zweckmäßig derart ausgestaltet, dass sie den Verdampfer im Verdampfungszustand zum Verdampfen der in der Aufnahmestruktur aufgenommenen Flüssigkeit mittels der Batterie elektrisch versorgt.

Der Inhalator kann prinzipiell zum Verdampfen beliebiger Flüssigkeiten zum Einsatz kommen. Insbesondere ist es möglich, den Inhalator zum Verdampfen von medizinischen Wirkstoffen enthaltenden Flüssigkeiten einzusetzen. Insbesondere erlauben die definierte und/oder kontrollierbare Menge der verdampften Flüssigkeit eine definierte und/oder kontrollierbare Dosierung der Wirkstoffzuführung zu einem Patienten.

Bei bevorzugten Ausführungsformen erfolgt im Füllzustand ein vollständiges Befüllen der Aufnahmestruktur mit der im Behälter bevorraten Flüssigkeit. Dies führt zu einer verbesserten Kontrolle der Menge der verdampften Flüssigkeit. Das vollständige Befüllen der Aufnahmestruktur kann prinzipiell durch ein ausreichend langes Verbleiben des Inhalators im Füllzustand realisiert sein. Alternativ oder zusätzlich ist es vorstellbar, die Steuereinrichtung zum Realisieren des vollständigen Befüllens der Aufnahmestruktur einzusetzen. Zu diesem Zweck ist die Steuereinrichtung entsprechend ausgestaltet.

Bevorzugt sind Ausführungsformen, bei denen das Verdampfen der Flüssigkeit in der Aufnahmestruktur lediglich außerhalb des Füllzustands und im Verdampfungszustand erfolgt. Somit erfolgt eine verbesserte Kontrolle der Menge der verdampften Flüssigkeit. Der Inhalator ist also diskontinuierlich betrieben, weil die Verdampfungseinrichtung nach dem Verdampften der in Aufnahmestruktur aufgenommenen Flüssigkeit kein Dampf mehr erzeugen kann. Erst im Füllzustand gelangt wieder Flüssigkeit in die Aufnahmestruktur und kann mit dem Verdampfer verdampft werden. Zu diesem Zweck ist es bevorzugt, wenn eine elektrische Versorgung des Verdampfers im Füllzustand unterbrochen ist. Die Steuereinrichtung ist zu diesem Zweck vorteilhaft derart ausgestaltet, dass sie die elektrische Versorgung des Verdampfers im Füllzustand unterbricht.

Vorstellbar ist auch ein kontinuierlicher Betrieb des Inhalators, bei welchem der Verdampfer im Füllzustand in der Aufnahmestruktur aufgenommene Flüssigkeit verdampft, wobei Flüssigkeit kontinuierlich in die Aufnahmestruktur nachgelangt.

Bei bevorzugten Ausführungsformen erfolgt ein Nachfüllen von Flüssigkeit in die Aufnahmestruktur erst dann, wenn die zuvor in der Aufnahmestruktur aufgenommene Flüssigkeit gänzlich verdampft ist. Dies ermöglicht es, die verdampfte Menge der Flüssigkeit einfach und zuverlässig zu kontrollieren. Zu diesem Zweck kann die Steuereinrichtung entsprechend ausgestaltet sein. Die Steuereinrichtung kann also derart ausgestaltet sein, dass sie ein Verstellen vom Verdampfungszustand in den Füllzustand verhindert, bis die in der Aufnahmestruktur aufgenommene Flüssigkeit vollständig verdampft ist. Das Verdampfen der in der Aufnahmestruktur aufgenommenen Flüssigkeit kann hierbei durch einen Verdampfungsvorgang oder durch mehrere Verdampfungsvorgänge erfolgen.

Alternativ ist es vorstellbar, die beim jeweiligen Verdampfungsvorgang verdampfte Menge der Flüssigkeit mittels einer entsprechenden Sensorik zu überwachen.

Das Verdampfen der in der Aufnahmestruktur aufgenommenen Flüssigkeit erfolgt bei ausreichend hohen Temperaturen der Aufnahmestruktur. Entsprechend ist die Verdampfereinrichtung, insbesondere der Verdampfer, ausgestaltet. Das heißt, dass der Verdampfer bei elektrischer Versorgung Wärme ausreichend hoher Temperaturen erzeugt, welche zum Verdampfen der in der Aufnahmestruktur aufgenommenen Flüssigkeit führen.

Ein vollständiges Verdampfen der in der Aufnahmestruktur aufgenommenen Flüssigkeit erfolgt hierbei dann, wenn die Aufnahmestruktur für eine vorgegebene und somit bekannte Dauer entsprechend geheizt wird. Mit anderen Worten, ein vollständiges Verdampfen der in der Aufnahmestruktur aufgenommenen Flüssigkeit ist dann erreicht, wenn der Verdampfer für eine vorgegebene Dauer betrieben ist und/oder für eine vorgegebene Dauer Temperaturen in einem vorgegebenen Bereich, nachfolgend auch als Betriebsbereich bezeichnet, erzeugt. Das vollständige Verdampfen der in der Aufnahmestruktur aufgenommenen Flüssigkeit kann dabei mittels der Steuereinrichtung kontrolliert und/oder überwacht sein. Die Steuereinrichtung ist also entsprechend ausgestaltet. Insbesondere ist es dabei ausreichend, wenn die Steuereinrichtung die Dauer des Betriebs des Verdampfers kontrolliert und/oder überwacht.

Prinzipiell können die Aufnahmestruktur und der Verdampfer der Verdampfereinrichtung separate Bestandteile der Verdampfereinrichtung sein.

Als vorteilhaft gelten Ausführungsformen, bei denen die Aufnahmestruktur zumindest teilweise, vorzugsweise gänzlich, Bestandteil des Verdampfers ist. Das heißt, dass der Verdampfer die Aufnahmestruktur zumindest teilweise, vorzugsweise gänzlich, aufweist. Hierdurch erfolgen die Aufnahme der Flüssigkeit und das Verdampfen der Flüssigkeit im Verdampfer. Mit anderen Worten, die Wärmeerzeugung zum Verdampfen der Flüssigkeit sowie die Aufnahme der Flüssigkeit erfolgen beide im Verdampfer. Somit weist die Aufnahmestruktur im Betrieb eine homogene Temperatur auf, so dass die in der Aufnahmestruktur aufgenommene Flüssigkeit homogen und gleichmäßig erhitzt und verdampft wird.

Zudem entspricht auf diese Weise die Temperatur des Verdampfers der Temperatur der Aufnahmestruktur. Dies führt zu einer vereinfachten Kontrolle der verdampften Menge der Flüssigkeit sowie zu einer verbesserten Effizienz des Inhalator.

Bevorzugt sind Ausführungsformen, bei denen der Verdampfer als eine die Aufnahmestruktur aufweisende und elektrisch leitende Keramik, nachfolgend auch als Verdampferkeramik bezeichnet, ausgebildet ist.

Die Verdampferkeramik dient also vorteilhaft zugleich dem Aufnehmen und Speichern der zu verdampfenden Flüssigkeit und dem Erzeugen von Wärme zum Verdampfen der Flüssigkeit. Das Speichern der Flüssigkeit erfolgt mittels der Aufnahmestruktur der Verdampferkeramik. Die Verdampferkeramik weist also die Aufnahmestruktur auf, in welcher im Betrieb die zu verdampfende Flüssigkeit aufgenommen ist.

Zur elektrischen Versorgung des Verdampfers, insbesondere der Verdampferkeramik, weist die Verdampfereinrichtung zwei elektrische Anschlüsse auf. Zwischen den beiden elektrischen Anschlüssen sowie durch den Verdampfer, insbesondere durch die Verdampferkeramik, verläuft ein Pfad des elektrischen Stroms, nachfolgend auch als Strompfad bezeichnet.

Bevorzugt ist der Verdampfer, insbesondere die Verdampferkeramik, derart ausgestaltet, dass er mittels ihrer elektrisch leitfähigen Eigenschaft im Betrieb bei elektrischer Versorgung im Verdampfer, insbesondere in der Verdampferkeramik, homogen Wärme erzeugt, um die in der Aufnahmestruktur aufgenommene Flüssigkeit zu verdampfen. Die homogene Wärmeerzeugung der Verdampferkeramik führt zu einer homogenen Temperatur bzw. einer homogenen Wärmeverteilung im Volumen der Verdampferkeramik und folglich in der Aufnahmestruktur. Dies führt zu einer gleichmäßigen Verdampfung der Flüssigkeit in der gesamten Aufnahmestruktur.

Der Verdampfer, insbesondere die Verdampferkeramik, ist für den Betrieb in einem thermischen Betriebsbereich ausgelegt, der von einer unteren Betriebsanfangstemperatur und einer oberen Betriebsendtemperatur begrenzt ist. Mit anderen Worten, zum Verdampfen der Flüssigkeit erzeugt der Verdampfer, insbesondere die Verdampferkeramik, bei elektrischer Versorgung Wärme im Betriebsbereich und somit zwischen der Betriebsanfangstemperatur und der Betriebsendtemperatur.

Die Kontrolle der Temperatur des Verdampfers, insbesondere der Verdampferkeramik, kann prinzipiell mittels einer entsprechenden Sensorik, welche zweckmäßig kommunizierend mit der Steuereinrichtung verbunden ist, erfolgen.

Als vorteilhaft gelten Ausführungsformen, bei denen der Inhalator, insbesondere die Verdampfereinrichtung, einen elektrischen Leiter aufweist, der nachfolgend auch als Sperrleiter bezeichnet wird. Der Sperrleiter ist im Strompfad angeordnet, sodass der elektrische Strom im Betrieb durch den Sperrleiter fließt. Der Sperrleiter ist dabei wärmeübertragend mit dem Verdampfer, insbesondere der Verdampferkeramik, verbunden. Zudem ist der Sperrleiter derart ausgestaltet, dass er bei der Betriebsendtemperatur einen sprunghaft ansteigenden elektrischen Widerstand aufweist. Durch die wärmeübertragende Verbindung des Sperrleiters mit der Verdampferkeramik erfolgt in der Folge beim Erreichen der Betriebsendtemperatur ein sprunghafter Anstieg des elektrischen Widerstands im Strompfad und somit eine Unterbrechung oder zumindest erhebliche Reduzierung der elektrischen Versorgung des Verdampfers, insbesondere der Verdampferkeramik. Dementsprechend wird die Betriebsendtemperatur mittels des Sperrleiters zumindest im Wesentlichen beeinflusst, vorzugsweise bestimmt.

Somit ist die Betriebsendtemperatur ohne zusätzliche Sensorik vorgegeben und bestimmt. Bei elektrischer Versorgung des Verdampfers, insbesondere der Verdampferkeramik, weist also der Verdampfer eine Temperatur zwischen der Betriebsanfangstemperatur und der Betriebsendtemperatur auf. Somit kann über die Dauer der elektrischen Versorgung des Verdampfers auf einfache und zuverlässige weiße die Menge der verdampften Flüssigkeit kontrolliert werden. Insbesondere ist es auf diese Weise möglich, dass vollständige Verdampfen der gesamten in der Aufnahmestruktur aufgenommenen Flüssigkeit über eine vorgegebene Dauer der elektrischen Versorgung des Verdampfers zu erreichen.

Die wärmeübertragende Verbindung des Sperrleiters mit dem Verdampfer, insbesondere der Verdampferkeramik, ist vorteilhaft derart, dass die Temperatur des Sperrleiters zumindest im Wesentlichen der Temperatur des Verdampfers, insbesondere der Verdampferkeramik, entspricht.

Der Inhalator, insbesondere die Verdampfereinrichtung, kann prinzipiell einen einzigen Sperrleiter aufweisen.

Vorstellbar ist es auch, der Inhalator, insbesondere die Verdampfereinrichtung, mit zwei oder mehr solchen Sperrleitern zu versehen. Bevorzugt ist es hierbei, wenn die Sperrleiter identisch sind.

Unter einem sprunghaften Anstieg des elektrischen Widerstands beim Überschreiten der Betriebsendtemperatur ist vorliegend ein derartiger Anstieg zu verstehen, der einen linearen Anstieg übersteigt.

Bevorzugt ist es, wenn zumindest einer der wenigstens einen Sperrleiter beim Überschreiten der Betriebsendtemperatur einen potenziellen Anstieg des elektrischen Widerstands zeigt. Besonders bevorzugt sind Ausführungsformen, bei denen zumindest einer der wenigstens einen Sperrleiter derart ausgestaltet ist, dass sein elektrischer Widerstand beim Überschreiten der Betriebsendtemperatur anfängt, exponentiell anzusteigen. Als vorteilhaft erweisen sich Ausführungsformen, bei denen der elektrische Widerstand zumindest eines der wenigstens einen Sperrleiter, vorteilhaft des jeweiligen Sperrleiters, in den auf die Betriebsendtemperatur folgenden 50°C um zumindest eine Zehnerpotenz steigt. Somit lassen sich die Verdampfungsparameter besonders einfach und effektiv kontrollieren.

Als vorteilhaft gelten Ausführungsformen, bei denen zumindest einer der wenigstens einen Sperrleiter, vorteilhaft der jeweilige Sperrleiter, als ein Kaltleiter ausgestaltet ist, wobei die Betriebsendtemperatur zwischen einer Anfangstemperatur und einer Endtemperatur des zumindest einen Kaltleiters liegt. Kaltleiter weisen eine charakteristische Strom-Kennlinie auf, wobei der elektrische Widerstand ab der Anfangstemperatur sprunghaft um mehrere Zehnerpotenzen steigt. Auf diese Weise wird also erreicht, dass der Sperrleiter den elektrischen Widerstand der gesamten Verdampfereinrichtung, nachfolgend auch als Gesamtwiderstand bezeichnet, bis zur Anfangstemperatur nicht oder möglichst geringfügig beeinflusst, und dass der Sperrleiter erst beim Erreichen der Anfangstemperatur einen den Gesamtwiderstand erhöhenden Einfluss hat. Mit anderen Worten, der Gesamtwiderstand wird auf diese Weise bis zum Erreichen der Betriebsendtemperatur vom Verdampfer, insbesondere von der Verdampferkeramik, und mit Erreichen der Betriebsendtemperatur von dem zumindest einen Sperrleiter dominiert. In der Folge kann der Betrieb im thermischen Betriebsbereich mit reduziertem Energieaufwand und somit erhöhter Effizienz erfolgen. Zugleich erfolgt beim Erreichen der Betriebsendtemperatur eine genau definierte und zuverlässige Unterbrechung oder zumindest Reduzierung der elektrischen Versorgung.

Prinzipiell kann die Betriebsendtemperatur beliebig zwischen der Anfangstemperatur und der Endtemperatur, vorteilhaft zwischen der Anfangstemperatur und der Nenntemperatur, des Kaltleiters liegen.

Vorteilhaft ist es, wenn die Betriebsendtemperatur der Anfangstemperatur des Kaltleiters entspricht. Somit wird insbesondere erreicht, dass zum Erreichen der Betriebsendtemperatur kein erhöhter Energieaufwand, insbesondere kein erhöhter Stromverbrauch, notwendig ist. Dies führt zu einer erhöhten Effizienz der Verdampfereinrichtung. Zudem lässt sich die Verdampfereinrichtung auf diese Weise mit Batterien, insbesondere wiederaufladbaren Batterien, einfach und mit einer erhöhten Betriebsdauer betreiben. Ferner führt dies dazu, dass der Sperrleiter im Betriebsbereich keine oder möglichst keine Wärme erzeugt. Somit erfolgt eine verbesserte Kontrolle über die Verdampfungsparameter. Ferner ist es auf diese Weise möglich, den Verdampfer, insbesondere die Verdampferkeramik, mit einer Leistung zu betreiben, welche ohne den Sperrleiter zu einer Überhitzung führen würde. Dadurch ist es ohne aufwendige Regelung möglich, den Verdampfer, insbesondere die Verdampferkeramik, schnell und mit hoher Leistung auf Temperaturen im Betriebsbereich zu bringen und im Betriebsbereich zu halten.

Der jeweilige zumindest eine Sperrleiter kann prinzipiell beliebig im Strompfad angeordnet sein, sofern er wärmeübertragend mit dem Verdampfer, insbesondre der Verdampferkeramik, verbunden ist.

Denkbar ist es insbesondere, zumindest einen der wenigstens einen Sperrleiter zwischen dem Verdampfer und einem der Anschlüsse anzuordnen. Dies erlaubt eine einfache und kompakte Ausbildung der Verdampfereinrichtung.

Prinzipiell kann die wärmeübertragende Verbindung zwischen dem jeweiligen Sperrleiter und dem Verdampfer, insbesondere der Verdampferkeramik, beliebig ausgestaltet sein.

Bevorzugt sind Ausführungsformen, bei denen zumindest einer der wenigstens einen Sperrleiter, vorteilhaft der jeweilige Sperrleiter, flächig auf dem Verdampfer, insbesondere der Verdampferkeramik, aufliegt. Insbesondere kann einer der wenigstens einen Sperrleiter unmittelbar flächig auf dem Verdampfer, insbesondere der Verdampferkeramik, aufliegen. Dies führt zu einer einfachen und kompakten Ausbildung der Verdampfereinrichtung, wobei zugleich eine einfache und zuverlässige Wärmeübertragung vom Verdampfer, insbesondere von der Verdampferkeramik, auf den Sperrleiter gegeben ist. Zugleich ist es auf diese Weise möglich, den Sperrleiter auf einfache Weise im Strompfad anzuordnen.

Der Verdampfer, insbesondere die Verdampferkeramik, ist vorteilhaft einstückig und zusammenhängend ausgebildet. Bevorzugt ist es hierbei, wenn an zumindest einer Außenseite des Verdampfers, insbesondere der Verdampferkeramik, ein Sperrleiter angeordnet ist. Somit ist eine kompakte und einfache Herstellung und Bauweise des Inhalators möglich.

Denkbar ist es auch, den Verdampfer, insbesondere die Verdampferkeramik, zwei- oder mehrteilig auszubilden. Der Verdampfer, insbesondere die Verdampferkeramik, kann also zwei voneinander separate Verdampferkörper aufweisen. Dabei kann zwischen wenigstens zwei der zumindest zwei Verdampferkörper ein Sperrleiter angeordnet sein.

Bevorzugt sind Ausführungsformen, bei denen der Verdampfer aus der Verdampferkeramik besteht, also ausschließlich die Verdampferkeramik aufweist. Dies führt zu einer vereinfachten Herstellung der Verdampfereinrichtung und zugleich einer genaueren und/oder einfachen Kontrolle der Verdampfungsparameter, insbesondere des Gesamtvolumens zur Aufnahme der zu verdampfenden Flüssigkeit und der erzeugten Wärme.

Der jeweilige zumindest eine Sperrleiter kann prinzipiell aus einem beliebigen Material bzw. Werkstoff hergestellt sein, sofern er beim Überschreiten der Betriebsendtemperatur einen sprunghaft ansteigenden elektrischen Widerstand aufweist.

Vorstellbar ist es insbesondere, dass zumindest einer der wenigstens einen Sperrleiter eine Keramik ist.

Zweckmäßig ist der zumindest eine Sperrleiter derart bemessen, dass er, im Vergleich zum Verdampfer, insbesondere zur Verdampferkeramik, volumenmäßig einen geringeren Anteil ausmacht. Dies erlaubt insbesondere eine kompaktere Ausbildung der Verdampfereinrichtung.

Bevorzugt sind Ausführungsformen, bei denen zumindest einer der wenigstens einen Sperrleiter als eine Lage ausgebildet ist. Insbesondere weist der zumindest eine Sperrleiter auf diese Weise ein im Vergleich zum Verdampfer, insbesondere zur Verdampferkeramik, erheblich reduziertes Volumen auf.

Die jeweilige Lage kann prinzipiell beliebig ausgestaltet sein. Insbesondere kann wenigstens eine der zumindest einen Lage als eine Folie, eine Beschichtung und dergleichen ausgestaltet sein.

Wie vorstehend beschrieben ist es bevorzugt, wenn der elektrische Gesamtwiderstand der Verdampfereinrichtung im thermischen Betriebsbereich vom Verdampfer, insbesondere von der Verdampferkeramik, und oberhalb des Betriebsbereichs, das heißt beim Überschreiten der Betriebsendtemperatur, vom Sperrleiter dominiert ist.

Bevorzugt ist dies derart realisiert, dass der elektrische Widerstand des Sperrleiters im Betriebsbereich maximal der Hälfte des elektrischen Widerstands des Verdampfers, insbesondere der Verdampferkeramik, entspricht.

Der elektrische Widerstand des zumindest einen Sperrleiters setzt sich insbesondere aus dem spezifischen Widerstand sowie dem Volumen bzw. der Strecke entlang des Strompfads zusammen. Dementsprechend kann eine Reduzierung des elektrischen Widerstands des Sperrleiters im Betriebsbereich durch eine Reduzierung des relativen Volumens des Sperrleiters im Inhalators, insbesondere in der Verdampfereinrichtung, erreicht werden.

Der Verdampfer, insbesondere die Verdampferkeramik, weist vorzugsweise einen elektrischen Widerstand auf, der bis zur Betriebsendtemperatur, insbesondere im Vergleich zum Anstieg des Widerstands des Sperrleiters ab der Betriebsendtemperatur, geringfügig ansteigt. Bevorzugt weist der elektrische Widerstand des Verdampfers, insbesondere der Verdampferkeramik, einen im Betriebsbereich temperaturabhängigen Verlauf derart auf, dass der Widerstand mit der Temperatur maximal um eine Zehnerpotenz steigt.

Die Verdampferkeramik erzeugt, wie erwähnt, bei elektrischer Versorgung mittels ihrer elektrischen Leitfähigkeit homogen Wärme. Insbesondere ist die Verdampferkeramik ein Heizwiderstand.

Die Verdampferkeramik kann eine elektrisch leitfähige Keramik beliebiger Art sein, sofern sie die Aufnahmestruktur aufweist und bei elektrische Versorgung, insbesondere beim Anliegen einer elektrischen Spannung in einem vorgegebenen Bereich, homogen Wärme im Betriebsbereich erzeugt.

Vorstellbar ist es, dass die Verdampferkeramik per se elektrisch leitfähig ist. Hierzu zählen beispielsweise Keramiken aus Metalloxiden, wie Titan-Oxide, oder Metallcarbide sowie Silizium-Carbide. Ebenso können Komposit-Keramiken zum Einsatz kommen, welche elektrisch leitfähige und elektrisch nicht leitfähige Netzwerke unterschiedlicher Werkstoffe aufweisen, wobei die leitfähigen Netzwerke zweckmäßig homogen in der Keramik verteilt sind. Beispiele für solche Komposit-Keramiken sind solche mit Metalloxiden unterschiedlicher Oxidationsstufe. Auch können Mischoxid-Keramiken zum Einsatz kommen, welche durch Vermengen unterschiedlicher Ausgangwerkstoffe hergestellt werden, wobei beim Herstellen der Keramik, typischerweise beim Sintern, durch chemische Reaktionen ein neuer Werkstoff entsteht. Beispiele für die Ausgangswerkstoffe sind unterschiedliche Metalloxide. Ferner können dotierte Keramiken zum Einsatz kommen, welche durch Dotierung elektrisch leitfähig werden. Selbstverständlich können auch beliebige Kombinationen der genannten Keramiken zum Einsatz kommen, sofern die Verdampferkeramik eine elektrische leitfähige Keramik mit der Aufnahmestruktur ist, welche im Betrieb homogen Wärme erzeugt.

Prinzipiell kann zum Verstellen des Inhalators zwischen dem Füllzustand und dem Verdampfungszustand eine Ventileinrichtung mit zumindest einem Ventil vorgesehen sein, welches die fluidische Verbindung zwischen dem Behälter und der Aufnahmestruktur entsprechend herstellt und trennt.

Erfindungsgemäß erfolgt das Verstellen des Inhalators zwischen dem Füllzustand und dem Verdampfungszustand durch eine relative Bewegung zwischen dem Behälter und der Verdampfereinrichtung, insbesondere der Aufnahmestruktur. Der jeweilige Zustand entspricht also einer relativen Stellung zwischen dem Behälter und der Aufnahmestruktur. Mit anderen Worten, der Füllzustand entspricht einer Füllstellung und der Verdampfungszustand einer Verdampfungsstellung. Auf diese Weise ist es insbesondere möglich, auf eine entsprechende Ventileinrichtung zu verzichten. Der Inhalator lässt sich auf diese Weise also vereinfacht und kostengünstig sowie gewichtsreduziert herstellen.

Bevorzugt sind Ausführungsformen, bei denen der Behälter einen Behälterauslass zum Auslassen der Flüssigkeit in die Aufnahmestruktur und somit Befüllen der Aufnahmestruktur mit der Flüssigkeit aufweist. Zudem weist die Aufnahmestruktur eine im Füllzustand mit dem Behälterauslass fluidisch verbundene Außenfläche auf, über welche Flüssigkeit über den Behälterauslass in die Aufnahmestruktur gelangt. Diese Außenfläche wird nachfolgend auch als Aufnahmefläche bezeichnet. Die Verdampfereinrichtung weist eine an der Aufnahmefläche anschließende Dichtung auf. Dabei grenzt die Aufnahmefläche im Füllzustand an den Behälterauslass an, sodass die Aufnahmestruktur fluidisch mit dem Behälter verbunden ist. Im Verdampfungszustand dichtet demgegenüber die Dichtung den Behälterauslass ab. Somit erfolgt ein einfaches Verstellen des Inhalators zwischen dem Füllzustand und dem Verdampfungszustand. Ferner wird somit auf einfache und zuverlässige Weise im Verdampfungszustand das Ausströmen von Flüssigkeit aus dem Behälterauslass vermieden.

Als vorteilhaft gelten Ausführungsformen, bei denen das Befüllen der Aufnahmestruktur mit der Flüssigkeit und die Ausgabe des erzeugten Dampfs aus der Aufnahmestruktur über zueinander beanstandeten Abschnitten der Außenfläche der Aufnahmestruktur erfolgt. Das heißt, dass die Aufnahmestruktur eine zur Aufnahmefläche beanstandete Außenfläche, nachfolgend auch als Ausgabefläche bezeichnet, aufweist, aus welcher im Betrieb bei elektrischer Versorgung des Verdampfers Dampf austritt. Somit erfolgt eine einfache Umsetzung des Inhalators bei zugleich verbesserter Kontrolle der verdampften Menge der Flüssigkeit.

Prinzipiell können die Aufnahmefläche und die Ausgabefläche der Aufnahmestruktur relativ zueinander beliebig positioniert sein, sofern sie zueinander beanstandet sind.

Denkbar ist es insbesondere, dass die Aufnahmefläche und die Ausgabefläche der Aufnahmestruktur voneinander abgewandt sind.

Als vorteilhaft gelten Ausführungsformen, bei denen der Behälter eine Innenkontur aufweist, welche den Behälterauslass aufweist. Dabei ist die Verdampfungseinrichtung zum Verstellen zwischen dem Füllzustand und dem Verdampfungszustand entlang der Innenkontur geführt. Somit ist der Inhalator einfach und kompakt ausgebildet. Zudem ist auf diese Weise das Verstellen des Inhalators zwischen dem Füllzustand und dem Verdampfungszustand auf einfache Weise umgesetzt.

Bevorzugt ist es, wenn der Behälter in der Art eines Zylinders und die Verdampfereinrichtung, insbesondere der Verdampfer, in der Art eines im Zylinder geführten Kolbens ausgebildet sind. Hierbei weist der Zylinder zweckmäßig eine Innenfläche auf, welche gemeinsam mit einer Außenfläche des Zylinders ein Volumen des Behälters zum Bevorraten der Flüssigkeit, nachfolgend auch als Behältervolumen bezeichnet, begrenzt. Die Verdampfungseinrichtung, insbesondere der Verdampfer, ist außerhalb des Behältervolumens und entlang der Innenfläche angeordnet und geführt. Vorteilhaft bildet dabei die Innenkontur die Innenfläche zumindest teilweise. Dies führt zu einer besonders einfachen Ausbildung des Inhalators und eine besonders einfache Verstellung zwischen dem Füllzustand und dem Verdampfungszustand.

Vorteilhaft ist es, wenn die Innenfläche des Behälters, insbesondere die Innenkontur, einen Kamin für die mit dem Verdampfer verdampfte Flüssigkeit bildet. Das heißt, dass der mit der Innenfläche begrenzte Querschnitt hin zu einem Auslass des Inhalators zur Ausgabe des Aerosols abnimmt. Insbesondere ist der Querschnitt konstant und nimmt in einem im Verdampfungszustand an die Verdampfereinrichtung folgenden Abschnitt ab. Insbesondere ist der Zylinder als ein solcher Kamin ausgebildet.

Die Aufnahmestruktur kann prinzipiell beliebig ausgebildet sein. Vorzugsweise ist die Aufnahmestruktur integral im Verdampfer, insbesondere in der Verdampferkeramik, ausgebildet und/oder ausgeformt.

Die Aufnahmestruktur ist bevorzugt homogen, insbesondere homogen in der Verdampferkeramik, verteilt.

Vorstellbar ist es, die Aufnahmestruktur durch nachträgliche Bearbeitung in die Verdampfereinrichtung, insbesondere in die Verdampferkeramik, einzubringen. Dabei ist es denkbar, in die Verdampfereinrichtung, insbesondere in die Verdampferkeramik, Kanäle, beispielsweise Mikrokanäle, auszuformen, welche Bestandteil der Aufnahmestruktur sind oder die Aufnahmestruktur bilden.

Bevorzugt weist die Aufnahmestruktur Poren, vorteilhaft in der Verdampferkeramik, auf. Besonders bevorzugt besteht die Aufnahmestruktur aus Poren, ist also eine Porenstruktur.

Die Poren der Verdampferkeramik sind vorteilhaft bei der Herstellung der Verdampferkeramik, welche beispielsweise mittels Sintern erfolgen kann, ausgebildet. Mit anderen Worten, die Poren zum Aufnehmen der Flüssigkeit sind vorteilhaft nicht gesondert, insbesondere nicht nachträglich, in die Verdampferkeramik eingebracht. Somit erfolgt die Verwendung einer intrinsischen, durch die Herstellung gegebenen, Eigenschaft der Verdampferkeramik zum Speichern der zu verdampfenden Flüssigkeit. Dies führt zu einer einfachen und kostengünstigen Herstellung der Verdampferkeramik und somit der Verdampfereinrichtung und des Inhalators.

Zudem lässt sich durch die Herstellung der Verdampferkeramik das Gesamtvolumen durch die Poren definieren. Dies führt zu einer weiteren, einfach ausgestalteten Kontrolle der Verdampfungsparameter.

Die Verdampfereinrichtung, insbesondere die Verdampferkeramik, kann prinzipiell Poren beliebiger Art aufweisen.

Vorteilhaft ist es, wenn die Verdampferkeramik Poren mit einer mittleren Größe zwischen 0,05 µm und 50 µm aufweist. Diese mittleren Porengrößen führen bei einer Flüssigkeit als Substanz zu einem derartigen Verhältnis zwischen der Oberfläche und dem Volumen der jeweiligen Pore, dass diese kapillaren Kräfte aufweisen, welche die gravitationsbedingt und/oder druckbedingt auf ein in dem Volumen aufgenommenes tropfenförmiges Teilchen der Flüssigkeit wirkenden Kräfte ausgleichen, vorzugsweise überwiegen. Daraus resultiert, dass die tropfenförmigen Teilchen, nachfolgend auch als Tröpfchen bezeichnet, in den Poren verbleiben. Folglich ist ein Abfließen der Tröpfchen und folglich der Flüssigkeit aus der Verdampferkeramik verhindert oder zumindest erheblich reduziert. Somit lassen sich in der Verdampferkeramik auch niederviskose Flüssigkeiten aufnehmen und speichern. Somit ist es mit der Verdampferkeramik also möglich, eine größere Variabilität an Flüssigkeiten unterschiedlicher Viskosität aufzunehmen und zu speichern, ohne dass die Flüssigkeiten aus der Verdampferkeramik abfließen. In der Folge können die Flüssigkeiten kostengünstiger und in einem breiteren Spektrum bereitgestellt werden. Insbesondere können somit in den Flüssigkeiten aufgenommene Wirkstoffe vereinfacht und/oder mit einer genaueren Dosis bereitgestellt werden. Somit lassen sich die Verdampferkeramik und die zugehörige Verdampfereinrichtung vereinfacht für eine kontrollierbare Inhalation besagter Wirkstoffe und somit eine kontrollierbare und/oder vorgegebene Dosierung der Wirkstoffe einsetzen. Die vorstehend beschriebenen kapillaren Kräfte führen ferner dazu, dass sich die Verdampferkeramik bei einer hydraulischen Verbindung mit der zur verdampfenden Flüssigkeit ohne weitere Einwirkung, wie beispielsweise ein aktives Pumpen der Flüssigkeit in die Verdampferkeramik, mit der Flüssigkeit vollsaugt. Insgesamt können somit gesonderte Abdichtungen der Verdampferkeramik entfallen oder zumindest reduziert werden und/oder Einrichtungen zum aktiven Einbringen der Flüssigkeit in die Keramik entfallen. Somit lassen sich sowohl die Verdampferkeramik als auch eine zugehörige Verdampfereinrichtung einfach und kostengünstig umsetzen. Somit erfolgt also neben einer Erhöhung der Einsatzmöglichkeiten der Verdampferkeramik sowie der zugehörigen Verdampfereinrichtung eine vereinfachte Umsetzung derselben.

Ein weiterer Vorteil der besagten mittleren Porengrößen ist darin zu sehen, dass diese zu einer Vergrößerung der mit der Verdampferkeramik in Kontakt stehenden Fläche der Tröpfchen führen. Mit anderen Worten, eine vergrößerte Fläche der Verdampferkeramik überträgt zum Verdampfen der Flüssigkeit Wärme auf die Tröpfchen. Dies führt zu einer gleichmäßigeren Verdampfung der Flüssigkeit und somit einer verbesserten Kontrolle über die Verdampfung. Zudem kommt es auf diese Weise zu einer schnelleren Verdampfung der Flüssigkeit.

Unter mittlere Porengröße ist vorliegend insbesondere das Verhältnis zwischen dem vierfachen Volumen und der Fläche der Poren, also 4V/A, wie dies insbesondere in der Norm ISO 15901 angegeben ist, zu verstehen.

Wie vorstehend beschrieben, kann die Verdampferkeramik insbesondere zur Aufnahme von niederviskosen Flüssigkeiten zum Einsatz kommen. Unter niederviskose Flüssigkeiten sind dabei insbesondere Flüssigkeiten zu verstehen, welche eine Viskosität von 45 mPas und kleiner aufweisen.

Bei der Flüssigkeit kann es sich um eine beliebige Flüssigkeit handeln. Insbesondere ist es möglich medizinische Wirkstoffe enthaltende Flüssigkeiten einzusetzen.

Bevorzugt ist es, wenn die Porengrößen der Poren der Porenstruktur zumindest größtenteils innerhalb der mittleren Porengröße liegen. Das heißt insbesondere, dass maximal 10 % der Poren Porengrößen aufweisen, welche größer sind als das 4-fache der mittleren Porengröße. Dies führt dazu, dass Poren mit Porengrößen oberhalb der mittleren Porengröße reduziert, vorzugsweise nicht, vorhanden sind. In der Folge sind die Auswirkungen von Poren mit Porengrößen oberhalb der mittleren Porengröße auf das Gesamtverhalten der Verdampferkeramik und folglich die Auswirkungen von Tröpfchen mit größeren Volumen in diesen Poren auf das Gesamtverhalten der in der Verdampferkeramik aufgenommenen Flüssigkeit vernachlässigbar oder zumindest reduziert. Somit lässt sich insbesondere verhindern, dass die Flüssigkeit aus der Verdampferkeramik abfließt. Dies führt ferner dazu, dass die in den Poren aufgenommenen Tröpfchen entsprechen der Größenverteilung der Poren im Wesentlichen gleiche Volumen aufweisen. Dies führt zu einer homogenen Verteilung der in der Verdampferkeramik aufgenommenen Flüssigkeit über das Volumen der Keramik. Darüber hinaus lässt sich die Flüssigkeit auf diese Weise homogener und/oder kontrollierter verdampfen.

Als vorteilhaft gelten Ausführungsformen, bei denen die mittlere Porengröße zwischen 0,1 µm und 25 µm, bevorzugt zwischen 0,15 µm und 10 µm, besonders bevorzugt zwischen 0,2 µm und 5 µm beträgt. Auf diese Weise kommt es zu einer vorteilhaften Wechselwirkung zwischen der in den Poren aufgenommenen Tröpfchen, den kapillaren Kräften sowie der Verteilung der Flüssigkeit im Volumen der Verdampferkeramik, welche zu einer verbesserten Aufnahme der Flüssigkeit in der Verdampferkeramik sowie einer verbesserten Verdampfung der in der Verdampferkeramik aufgenommenen Flüssigkeit führen.

Die Verdampfungseinrichtung und/oder der Behälter können jeweils fester Bestandteil des Inhalators oder im Inhalator austauschbar angeordnet sein.

Vorstellbar ist es, dass die Verdampfungseinrichtung und der Behälter fester Bestandteil des Inhalators sind, wobei der Behälter mit Flüssigkeit nachfühlbar ist.

Denkbar ist es, dass die Verdampfereinrichtung fester Bestandteil des Inhalators ist, wogegen der Behälter im Inhalator austauschbar aufgenommen ist.

Bei bevorzugten Ausführungsformen bilden der Behälter und die Verdampfungseinrichtung eine im Inhalator austauschbar aufgenommene Einheit, insbesondere in der Art einer Kapsel. Dies führt zu einer vereinfachten Handhabung des Inhalators. Zudem sind auf diese Weise durch eine entsprechend klare Zuordnung jeweils eines Behälters zu einer zugehörigen Verdampfereinrichtung Querkontaminationen von unterschiedlichen Flüssigkeiten vermieden.

Bevorzugt ist es hierbei, wenn der Behälter abgeschlossen, also nicht zerstörungsfrei mit Flüssigkeit nachfüllbar, ist. Dies führt insbesondere zur Vermeidung von Querkontaminationen von unterschiedlichen Flüssigkeiten und/oder verhindert die Verwendung nicht vorgegebener und/oder nicht zugelassener Flüssigkeiten, oder erschwert diese Verwendung zumindest.

Vorteilhaft ist die Steuereinrichtung mit der Verdampfereinrichtung und/oder mit dem Behälter derart kommunizierend verbunden, dass die Steuereinrichtung die im Behälter bevorratete bzw. in der Aufnahmestruktur aufgenommene Flüssigkeit übermittelt bekommt und/oder erkennt. Somit ist es insbesondere möglich, für unterschiedliche Flüssigkeiten eine zugehörige elektrische Versorgung der Verdampfereinrichtung vorzunehmen. Ferner ist es somit möglich, das Verdampfen von nicht zugelassenen und/oder freigegebenen Flüssigkeiten zu verhindern. Ebenso ist es somit möglich, einen Betrieb des Inhalators, insbesondere eine elektrische Versorgung der Verdampfungseinrichtung zum Verdampfen der Flüssigkeit, lediglich dann zuzulassen, wenn vorgegebene und/oder zugelassene Behälter und/oder Verdampfungseinrichtungen im Inhalators aufgenommen sind. Entsprechend ist die Steuereinrichtung ausgestaltet. Somit ist es also insbesondere möglich, einen Missbrauch des Inhalators zu verhindern.

Die Kommunikation zwischen der Steuereinrichtung und dem Behälter und/oder der Verdampfereinrichtung ist vorteilhaft über entsprechende Kommunikationsschnittstellen realisiert. Die Steuereinrichtung weist also eine Steuereinrichtung-Kommunikationsschnittstelle auf, die kommunizierend mit einer Behälter-Kommunikationsschnittstelle des Behälters und/oder mit einer Verdampfer-Kommunikationsschnittstelle der Verdampfereinrichtung verbunden ist.

Die Kommunikation kann hierbei beliebig erfolgen. Vorstellbar ist eine kabelgebundene Kommunikation. Bevorzugt ist eine kabellose Kommunikation.

Bevorzugt sind Ausführungsformen, bei denen die Einheit mit dem Behälter und der Verdampfereinrichtung eine gemeinsame Kommunikationsschnittstelle zur Kommunikation mit der Steuereinrichtung-Kommunikationsschnittstelle aufweist.

Es versteht sich, dass neben dem Inhalator auch die den Behälter und die Verdampfereinrichtung umfassende Einheit als solche zum Umfang dieser Erfindung gehört.

Weitere wichtige Merkmale und Vorteile der Erfindung ergeben sich aus den Unteransprüchen, aus den Zeichnungen und aus der zugehörigen Figurenbeschreibung anhand der Zeichnungen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Komponenten beziehen.
- Fig. 1: eine stark vereinfachte, schaltplanartige Darstellung eines Inhalators,
- Fig. 2: eine isometrische Ansicht einer Verdampfereinrichtung des Inhalators,
- Fig. 3: eine isometrische Ansicht der Verdampfereinrichtung bei einem anderen Ausführungsbeispiel,
- Fig. 4: einen vereinfachten Schnitt durch den Inhalator in einem Füllzustand,
- Fig. 5: einen vereinfachten Schnitt durch den Inhalator in einem Verdampfungszustand.

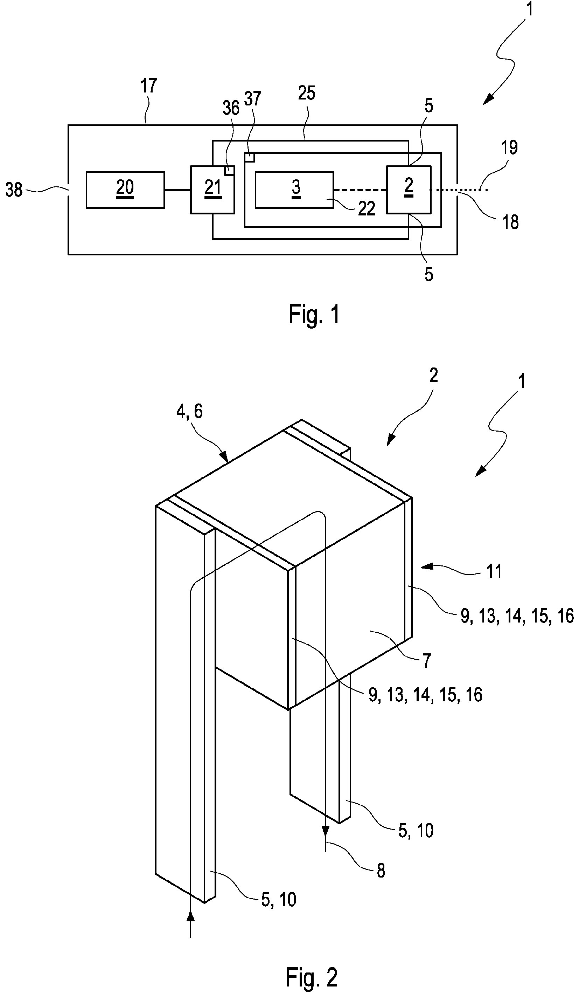

Ein Inhalator 1, wie er beispielsweise in den Figuren 1 bis 5 gezeigt ist, dient dem Inhalieren eines Aerosols. Zu diesem Zweck weist der Inhalator 1 eine Verdampfereinrichtung 2 sowie einen Behälter 3 auf. Der Behälter 3 dient dem Bevorraten einer zu verdampfenden Flüssigkeit. Mit der Verdampfereinrichtung 2 erfolgt das Verdampfen der Flüssigkeit zwecks Erzeugens des zu inhalierenden Aerosols.

Bei dem Inhalator 1 der gezeigten Ausführungsbeispiele handelt es sich um einen mobilen und händisch tragbaren Inhalator 1, der im Gebrauch von einem nicht gezeigten Nutzer händisch umgriffen und tragbar ist. Entsprechend ist der Inhalator 1 hinsichtlich seiner Abmessungen und seines Gewichts ausgebildet.

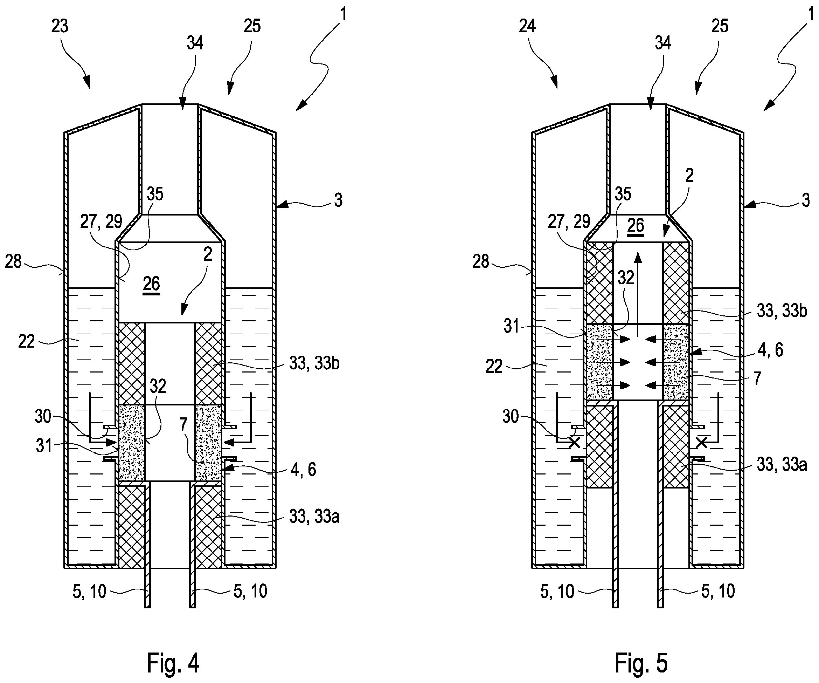

Die Verdampfereinrichtung 2 dient dem Verdampfen einer Flüssigkeit, insbesondere einer vorgegebenen Dosis der Flüssigkeit. Bei der Flüssigkeit handelt es sich beispielsweise um eine solche, welche einen medizinischen Wirkstoff beinhalten kann, sodass beim Verdampfen ein den Wirkstoff enthaltender Dampf 19 (s. Figur 1 und Figur 5) ausgegeben wird, der von einem Nutzer inhaliert wird.

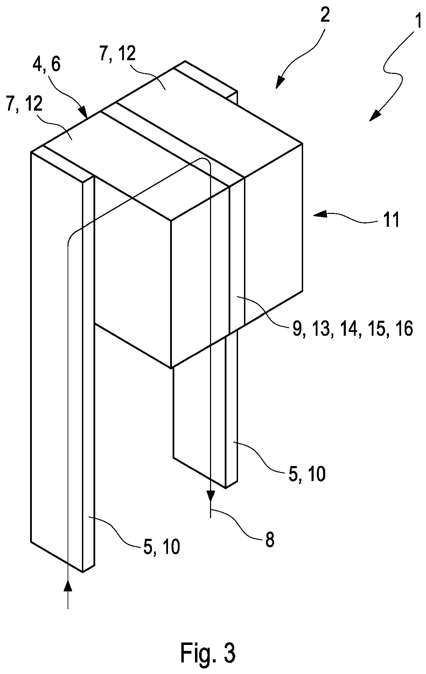

Wie den Figuren 2 und 3 entnommen werden kann, weist die Verdampfereinrichtung 2 einen elektrischen Verdampfer 4 zum Verdampfen der Flüssigkeit auf. Zur elektrischen Versorgung des Verdampfers 4 sind in den gezeigten Ausführungsbeispielen zwei elektrische Anschlüsse 5 vorgesehen. Die Verdampfereinrichtung 2 weist ferner eine Aufnahmestruktur 7 zum Aufnehmen von Flüssigkeit auf. Die Aufnahmestruktur 7 weist ein vorgegebenes und somit bekanntes Gesamtvolumen zum Aufnahmen von Flüssigkeit auf.

In den gezeigten Ausführungsbeispielen dient der Verdampfer 4 sowohl der Aufnahme und dem Speichern der zu verdampfenden Flüssigkeit als auch der Erzeugung von Wärme zwecks Verdampfens der Flüssigkeit. Zu diesem Zweck weist der Verdampfer 4 der gezeigten Ausführungsbeispiele eine elektrisch leitfähige Keramik 6 auf, welche nachfolgend auch als Verdampferkeramik 6 bezeichnet wird. In den gezeigten Ausführungsbeispielen besteht der Verdampfer 4 aus der Verdampferkeramik 6.

Die Aufnahmestruktur 7 der gezeigten Ausführungsbeispiele weist Poren (nicht gezeigt) der Verdampferkeramik 6 auf, sodass die zu verdampfende Flüssigkeit in den Poren aufgenommen wird. Vorteilhaft besteht die Aufnahmestruktur 7 aus dem Poren, ist also eine Porenstruktur. Die Aufnahmestruktur 7 besteht also vorteilhaft aus Poren der Verdampferkeramik 6. Die Verdampferkeramik 6 kann zumindest ein Metalloxid enthalten. Zum Verdampfen der Flüssigkeit ist die Verdampferkeramik 6 in einem thermischen Bereich, der nachfolgend auch als Betriebsbereich bezeichnet wird, betrieben. Der Betriebsbereich wird von einer niedrigen Temperatur, nachfolgend auch als Betriebsanfangstemperatur bezeichnet, und von einer hohen Temperatur, nachfolgend auch als Betriebsendtemperatur bezeichnet, begrenzt. Das heißt, dass das Verdampfen der zu verdampfenden und in den Poren aufgenommenen Flüssigkeit im Betriebsbereich und somit zwischen der Betriebsanfangstemperatur und der Betriebsendtemperatur erfolgt.

Zum Erzeugen von Wärme wird der Verdampfer 4 mittels der Anschlüsse 5 elektrisch versorgt, sodass ein in den Figuren 2 und 3 angedeuteter Pfad 8 des elektrischen Stroms zwischen den Anschlüssen 5 und durch den Verdampfer 4 führt, wobei der Pfad 8 nachfolgend auch als Strompfad 8 bezeichnet wird. Bei elektrischer Versorgung erzeugt die Verdampferkeramik 6 mittels ihres elektrischen Widerstands Wärme zum Verdampfen der Flüssigkeit.

Der Inhalator 1 weist ein lediglich in Figur 1 gezeigtes Gehäuse 17 auf, in welchem die Verdampfereinrichtung 2 aufgenommen ist, und welches eine Auslassöffnung 18 zum Auslassen des mittels des Verdampfers 4 erzeugten Dampfs 19 bzw. Aerosols aufweist. Ferner weist der Inhalator 1, insbesondere das Gehäuse 17, eine Einlassöffnung 38 zum Einlassen von Luft in den Inhalator 1 auf. Zudem ist der Behälter 3 im Gehäuse 17 aufgenommen. Der Inhalator 1 weist ferner eine, vorzugsweise wiederaufladbare, lediglich in Figur 1 gezeigte Batterie 20 zur elektrischen Versorgung der Verdampfereinrichtung 2 auf, welche im Gehäuse 17 aufgenommen ist.

Der Behälter 17 weist ein Volumen 22 zum Bevorraten von Flüssigkeit auf, welches nachfolgend auch als Behältervolumen 22 bezeichnet wird.

Der Inhalator 1 ist zwischen einem in Figur 4 gezeigten Füllzustand 23 und einem in Figur 5 gezeigten Verdampfungszustand 24 verstellbar. Im Füllzustand 23 ist die Aufnahmestruktur 7 fluidisch mit dem Behältervolumen 22 verbunden, sodass im Behältervolumen 22 bevorratete Flüssigkeit in die Aufnahmestruktur 7 gelangt und die Aufnahmestruktur 7 füllt. Vorzugsweise erfolgt im Füllzustand 23 ein vollständiges Befüllen der Aufnahmestruktur 7 mit der Flüssigkeit. Im Verdampfungszustand 24 ist die Aufnahmestruktur 7 demgegenüber fluidisch vom Behältervolumen 22 getrennt, sodass keine Flüssigkeit aus dem Behälter 3 in die Aufnahmestruktur 7 gelangt.

Das Verdampfen der Flüssigkeit erfolgt bevorzugt lediglich im Verdampfungszustand 24. Das heißt, dass der Verdampfer 4, in den gezeigten Ausführungsbeispielen die Verdampferkeramik 6, lediglich im Verdampfungszustand 24 Wärme zum Verdampfen der in der Aufnahmestruktur 7 aufgenommenen Flüssigkeit erzeugt. Zu diesem Zweck weist der Inhalator 1 eine lediglich in Figur 1 gezeigte Steuereinrichtung 21 auf, welche entsprechend ausgestaltet ist. Die Steuereinrichtung 21 versorgt also den Verdampfer 4 im Verdampfungszustand 24 elektrisch, sodass die in der Aufnahmestruktur 7 aufgenommene Flüssigkeit verdampft. Vorzugsweise wird zudem die elektrische Versorgung des Verdampfers 4, insbesondere der Verdampferkeramik 6, im Füllzustand 23 unterbrochen. Die Steuereinrichtung 21 ist derart mit der Batterie 20 verbunden, dass sie die elektrische Verbindung der Batterie 20 mit der Verdampfereinrichtung 2 zwecks elektrischer Versorgung der Verdampfereinrichtung 2 herstellen und unterbrechen kann.

Die Versorgung des Verdampfers 4 kann dabei derart erfolgen, dass die in der Aufnahmestruktur 7 aufgenommene Flüssigkeit gänzlich verdampft. Alternativ kann die Versorgung des Verdampfers 4 derart erfolgen, dass ein Teil der in der Aufnahmestruktur 7 aufgenommenen Flüssigkeit verdampft. In diesem Fall wird die Flüssigkeit in mehreren Schritten verdampft. In jedem Fall ist es bevorzugt, wenn die Aufnahmestruktur 7 nach dem vollständigen Verdampfen der zuvor darin aufgenommenen Flüssigkeit, also im trockenen Zustand, mit Flüssigkeit befüllt wird. Das heißt, dass der Inhalator 1 dann in den Füllzustand 23 verstellt wird oder werden kann, wenn die in der Aufnahmestruktur 7 aufgenommene Flüssigkeit gänzlich verdampft wurde. Dies kann mittels der Steuereinrichtung 21 realisiert sein, welche ein Verstellen vom Verdampfungszustand 24 in den Füllzustand 23 verhindert, bis die in der Aufnahmestruktur 7 aufgenommene Flüssigkeit vollständig verdampft ist.

In den gezeigten Ausführungsbeispielen ist, wie den Figuren 2 und 3 entnommen werden kann, im Strompfad 8 zumindest ein Sperrleiter 9 angeordnet, derart, dass der Strompfad 8 zwingend durch den Sperrleiter 9 führt. Dabei ist der Sperrleiter 9 lediglich in den Figuren 2 und 3 gezeigt und dargestellt. In den gezeigten Ausführungsbeispielen ist dies dadurch erreicht, dass der zumindest eine Sperrleiter 9 zwischen den Anschlüssen 5 angeordnet ist. Der zumindest eine Sperrleiter 9 ist dabei wärmeübertragend mit der Verdampferkeramik 6 verbunden. In den gezeigten Ausführungsbeispielen ist die wärmeübertragende Verbindung des zumindest einen Sperrleiters 9 mit der Verdampferkeramik 6 durch eine flächige Anordnung des Sperrleiters 9 auf der Verdampferkeramik 6 realisiert. Insbesondere liegt der Sperrleiter 9 unmittelbar an der Verdampferkeramik 6 an. Somit entspricht die Temperatur des zumindest einen Sperrleiters 9 der Temperatur der Verdampferkeramik 6. Der zumindest eine Sperrleiter 9 ist derart ausgestaltet, dass er beim Überschreiten der Betriebsendtemperatur einen sprunghaft ansteigenden elektrischen Widerstand aufweist. Unterhalb der Betriebsendtemperatur ist der zumindest eine Sperrleiter 9 also elektrisch leitend, sodass die Verdampferkeramik 6 bei elektrischer Versorgung im Betriebsbereich betrieben ist, das heißt Temperaturen bis zur Betriebsendtemperatur erreicht. Der sprunghafte Anstieg des elektrischen Widerstands des zumindest einen Sperrleiters 9 führt dazu, dass der durch die Verdampferkeramik 6 strömende elektrische Strom beim Überschreiten der Betriebsendtemperatur unterbrochen oder erheblich reduziert wird, sodass der sprunghafte Anstieg des elektrischen Widerstands des zumindest einen Sperrleiters 9 die Betriebsendtemperatur definiert oder zumindest dominiert. Somit ist es möglich, die Verdampfereinrichtung 2 mit kontrollierten Verdampfungsparametern zu betreiben. Dies erlaubt es insbesondere, eine vorgegebene Menge der zu verdampfenden Flüssigkeit und somit eine vorgegebene Dosis der Flüssigkeit zu verdampfen. Dabei ist hierzu eine separate Elektronik (nicht gezeigt) und/oder separate Sensorik (nicht gezeigt), beispielsweise zum Ermitteln der Temperatur der Verdampferkeramik 6, nicht notwendig. Dementsprechend weist der Inhalator 1 der gezeigten Ausführungsbeispiele vorteilhaft keine solche Sensorik und Elektronik auf.

Wie insbesondere den Figuren 2 und 3 entnommen werden kann, sind in den gezeigten Ausführungsbeispielen die elektrischen Anschlüsse 5 jeweils als eine Leiterplatte 10, beispielsweise aus einem Metall oder einer Metalllegierung, ausgebildet.

In den in den Figuren 2 und 3 gezeigten Ausführungsbeispielen ist der Verdampfer 4 zwischen den Anschlüssen 5 angeordnet. Im in den Figuren 4 und 5 gezeigten Ausführungsbeispiel sind beide Anschlüsse 5 an derselben Stirnseite des Verdampfers 4 angeordnet.

In den gezeigten Ausführungsbeispielen bilden der Verdampfer 4, insbesondere die Verdampferkeramik 6, und der zumindest eine Sperrleiter 9 ein zusammenhängendes Modul 11, welches zwischen den Anschlüssen 5 angeordnet ist. In den in den Figuren 2 und 3 gezeigten Ausführungsbeispielen weist das Modul 11 eine quaderförmige Ausbildung auf.

Beim in Figur 2 gezeigten Ausführungsbeispiel ist die Verdampferkeramik 6 zusammenhängend und quaderförmig ausgebildet, wobei zwischen der jeweiligen, einem der Anschlüsse 4 zugewandten Außenseite der Verdampferkeramik 6 und dem zugehörigen Anschluss 5 ein Sperrleiter 9 angeordnet ist.

Das in Figur 3 gezeigte Ausführungsbeispiel unterscheidet sich von dem in Figur 1 gezeigten Ausführungsbeispiel dadurch, dass die Verdampferkeramik 6 zweiteilig ausgebildet ist und somit zwei Verdampferkörper 12 aufweist, welche im gezeigten Ausführungsbeispiel jeweils identisch und quaderförmig ausgebildet sind. Hierbei ist in dem gezeigten Ausführungsbeispiel ein einziger Sperrleiter 9 vorgesehen, der zwischen den Verdampferkörpern 12 angeordnet ist.

Beim in den Figuren 4 und 5 gezeigten Ausführungsbeispiel ist der Verdampfer 4, insbesondere die Verdampferkeramik 6, ringförmig ausgebildet. Hierbei ist der Verdampfer 4 einteilig ausgebildet.

Wie insbesondere den Figuren 1 und 3 entnommen werden kann, ist in den gezeigten Ausführungsbeispielen ein Volumenanteil der Verdampferkeramik 6 im Gesamtvolumen des Moduls 11 erheblich größer als ein Volumenanteil des zumindest einen Sperrleiters 9. Insbesondere ist der Volumenanteil des zumindest einen Sperrleiters 9, nachfolgend auch als Sperrvolumen bezeichnet, maximal 1/10 des Volumenanteils der Verdampferkeramik 6, nachfolgend auch als Verdampfervolumen bezeichnet. Dies führt insbesondere dazu, dass das Gesamtvolumen zum Aufnehmen der Flüssigkeit von der Verdampferkeramik 6 bestimmt oder zumindest dominiert ist. Zudem führt dies dazu, dass der zumindest eine Sperrleiter 9 im Betriebsbereich eine vernachlässigbare Rolle beim elektrischen Gesamtwiderstand des Moduls 11 und somit des Verdampfers 4 spielt. Mit anderen Worten, der elektrische Gesamtwiderstand des Verdampfers 4 wird im Betriebsbereich von der Verdampferkeramik 6 dominiert, wohingegen er oberhalb des Betriebsbereichs von dem zumindest einen Sperrleiter 9 dominiert wird.

In den gezeigten Ausführungsbeispielen ist der jeweilige Sperrleiter 9 als eine im Vergleich zur Verdampferkeramik 6 bzw. zu den Verdampferkörpern 12 dünne Lage 13 ausgebildet und kann daher auch als Sperrlage 14 bezeichnet werden.

Der jeweilige Sperrleiter 9 ist vorzugsweise ein Kaltleiter 15, welcher ab einer Anfangstemperatur einen sprunghaften und um mehrere Zehnerpotenzen steigenden elektrischen Widerstand aufweist. Hierbei entspricht die Betriebsendtemperatur vorteilhaft einer Temperatur zwischen der Anfangstemperatur und einer Endtemperatur des Kaltleiters 15, insbesondere der Anfangstemperatur des Kaltleiters 15.

Insbesondere ist der Kaltleiter 15 eine sich von der Verdampferkeramik 6 unterscheidende Keramik 16, welche nachfolgend auch als Sperrkeramik 16 bezeichnet wird. Durch das geringere Sperrvolumen der Sperrkeramik 16 im Vergleich zum Verdampfervolumen der Verdampferkeramik 6 wird dabei die Gesamtaufnahmefähigkeit des Verdampfers 4 von der Verdampferkeramik 6 bestimmt oder zumindest dominiert.

In den gezeigten Ausführungsbeispielen und bevorzugt bilden die Verdampfereinrichtung 2 und der Behälter 3 eine Einheit 25, welche austauschbar im Inhalator 1, insbesondere im Gehäuse 17, aufgenommen ist. Der Behälter 3 der gezeigten Ausführungsbeispiele ist abgeschlossen. Das heißt, dass der Behälter 3 nicht mit Flüssigkeit nachgefüllt werden kann, ohne dass der Behälter 3 beschädigt, beispielsweise aufgebohrt, gebrochen und dergleichen, wird.

Alternativ ist es möglich, dass der Behälter 3 im Inhalator 1 fest aufgenommen und nachfüllbar ist. In diesem Fall kann auch die Verdampfereinrichtung 2 dauerhaft im Inhalator 1 aufgenommen sein.

Alternativ ist es möglich, dass der Behälter 3 austauschbar ist. In diesem Fall kann auch die Verdampfereinrichtung 2 dauerhaft im Inhalator 1 aufgenommen sein.

Der Inhalator 1 der gezeigten Ausführungsbeispiele ist derart ausgestaltet, dass die Steuereinrichtung 21 mit dem Behälter 3 und/oder der Verdampfereinrichtung 2 kommuniziert, um insbesondere die zu verdampfende Flüssigkeit und/oder die aufgenommene Einheit 25 zu erkennen und/oder übermittelt zu bekommen. Zu diesem Zweck weist die Steuereinrichtung 21 eine lediglich in Figur 1 gezeigte Kommunikationsschnittstelle 36 auf, welche nachfolgend auch als Steuereinrichtung-Kommunikationsschnittstelle 36 bezeichnet wird. In den gezeigten Ausführungsbeispielen weist ferner die Einheit 25 eine Kommunikationsschnittstelle 37 auf, welche nachfolgend auch als Einheit-Kommunikationsschnittstelle 37 bezeichnet wird. Steuereinrichtung-Kommunikationsschnittstelle 36 und Einheit-Kommunikationsschnittstelle 37 sind beim in Inhalator 1 aufgenommenen Zustand der Einheit 25 kommunizierend, vorzugsweise kabellos kommunizierend, miteinander verbunden. Dabei sind die Einheit-Kommunikationsschnittstelle 37 und die Steuereinrichtung 21, insbesondere die Steuereinrichtung-Kommunikationsschnittstelle 36, derart ausgestaltet, dass die Steuereinrichtung 21 die Einheit 25 und/oder die Flüssigkeit erkennt und/oder übermittelt bekommt. Zu diesem Zweck kann die Einheit-Kommunikationsschnittstelle 37 entsprechende Informationen beinhalten. Ist keine zugelassene Einheit 25 und/oder keine Einheit 25 einer vorgegebenen Art im Inhalator 1 aufgenommen, ist die Steuereinrichtung 21 zweckmäßig derart ausgestaltet, dass sie einen Betrieb des Inhalators 1 verhindert. Ebenso kann die Steuereinrichtung 21 derart ausgestaltet sein, dass sie einen Betrieb des Inhalators 1 bei einer fehlenden Kommunikation mit der Einheit 25 verhindert.

In den gezeigten Ausführungsbeispielen erfolgt das Verstellen des Inhalators 1 zwischen dem Füllzustand 23 und dem Verdampfungszustand 24 durch eine relative Bewegung zwischen der Verdampfungseinrichtung 2, insbesondere der Verdampferkeramik 6, und dem Behälter 3. In den gezeigten Ausführungsbeispielen erfolgt diese relative Bewegung translatorisch bzw. linear. Vorstellbar sind auch rotatorische relative Bewegungen zwischen der Verdampfereinrichtung 2 und dem Behälter 3. In den gezeigten Ausführungsbeispielen erfolgt die relative Bewegung zwischen der Verdampfereinrichtung 2, insbesondere der Verdampferkeramik 6, und dem Behälter 3 durch eine relative Bewegung der Verdampfereinrichtung 2 zum Behälter 3.

In dem in den Figuren 4 und 5 gezeigten Ausführungsbeispiel weist der Behälter 3 eine zylinderartige Form mit einer einen Hohlraum 26 begrenzenden Innenfläche 27 und einer von der Innenfläche 27 abgewandten Außenfläche 28 auf. Die Innenfläche 27 und die Außenfläche 28 begrenzen das Behältervolumen 22. Die Verdampfereinrichtung 2 ist im Hohlraum 26 angeordnet und entlang der Innenfläche 27 kolbenartig geführt und somit verstellbar. Zu diesem Zweck weist die Innenfläche 27 eine entsprechende Innenkontur 29 auf. Der Behälter 3 weist an der Innenfläche 27, insbesondere der Innenkontur 29, einen offenen Auslass 30, nachfolgend auch als Behälterauslass 30 bezeichnet, auf. Der Behälterauslass 30 dient dem Auslassen der im Behältervolumen 22 bevorraten Flüssigkeit in die Aufnahmestruktur 7. Die Aufnahmestruktur 7 weist hierbei eine der Innenfläche 27 zugewandte Fläche 31 auf, über welche im Füllzustand 23 Flüssigkeit über den Behälterauslass 30 in die Aufnahmestruktur 7 gelangt. Im gezeigten Ausführungsbeispiel weist die Aufnahmestruktur 7 zu dieser Fläche 31, welche nachfolgend auch als Aufnahmefläche 31 bezeichnet wird, beanstandet eine Fläche 32 zur Ausgabe der im Verdampfungszustand 24 verdampften Flüssigkeit auf. Diese Fläche 32 wird nachfolgend auch als Ausgabefläche 32 bezeichnet. In dem in den Figuren 4 und 5 gezeigten Ausführungsbeispiel sind die Aufnahmefläche 31 und die Ausgabefläche 32 voneinander abgewandt. Die Ausgabefläche 32 ist also auf der von der Innenfläche 27 des Behälters 3 abgewandten Seite der Aufnahmefläche 31 angeordnet. Die Ausgabefläche 32 ist hin zum Hohlraum 26 offen. Die Aufnahmefläche 31, vorzugsweise die Aufnahmestruktur 7, ist im Verdampfungszustand 24 zum Behälterauslass 30 beanstandet. Die Verdampfereinrichtung 1 weist ferner eine Dichtung 33 auf, welche an einer Stirnseite der Aufnahmestruktur 7 bzw. der Verdampferkeramik 6 angeordnet ist. Diese Dichtung 33, welche nachfolgend auch als erste Dichtung 33a bezeichnet wird, verschließt im Verdampfungszustand 24 den Behälterauslass 30 und dichtet den Behälterauslass 30 fluidisch ab. Demgegenüber grenzt im Füllzustand 23 die Aufnahmefläche 31 an den Behälterauslass 30.

In dem in den Figuren 4 und 5 gezeigten Ausführungsbeispiel weist die Verdampfereinrichtung 2 eine auf der von der ersten Dichtung 33a abgewandten Seite der Aufnahmestruktur 7 bzw. der Verdampferkeramik 6 stirnseitig angeordnete weitere Dichtung 33 auf, welche nachfolgend auch als zweite Dichtung 33b bezeichnet wird. Die zweite Dichtung 33b verhindert insbesondere den Austritt von Dampf aus der Stirnseite der Aufnahmestruktur 7, welche sie überdeckt. Beide Dichtungen 33 verhindern ferner, dass Flüssigkeit über den Behälterauslass 30 direkt in den Hohlraum 26 gelangt.

In dem in den Figuren 4 und 5 gezeigten Ausführungsbeispiel bildet der Behälter 3, insbesondere die Innenfläche 27, einen Kamin 34 für die verdampfte Flüssigkeit. Zu diesem Zweck weist der Behälter 3 einen durchströmbaren Querschnitt auf, welcher hin zur Auslassöffnung 18 abnimmt. Im gezeigten Ausführungsbeispiel weist der Behälter 3 dabei ein bis zu einem Anschlag 35 konstanten Querschnitt auf, der ab dem Anschlag 35 hin zur Auslassöffnung 18 verkleinert ist. Wie insbesondere Figur 5 entnommen werden kann, bildet der Anschlag 35 im gezeigten Ausführungsbeispiel eine Begrenzung für die Verdampfereinrichtung 2 im Verdampfungszustand 24. Hierbei schlägt die zweite Dichtung 33b im Verdampfungszustand 24 am Anschlag 35 an.

## Patentansprüche

1. Inhalator (1) zum Inhalieren eines Aerosols,
- mit einem Behälter (3) zum Bevorraten einer Flüssigkeit,
- mit einer Verdampfereinrichtung (2), die einen elektrischen Verdampfer (4) zum Verdampfen der Flüssigkeit und somit Erzeugen des Aerosols aufweist,
- wobei die Verdampfereinrichtung (2) eine Aufnahmestruktur (7) zur Aufnahme der Flüssigkeit aufweist,
- wobei der Inhalator (1) zwischen einem Füllzustand (23) und einem Verdampfungszustand (24) verstellbar ist,
- wobei die Aufnahmestruktur (7) und der Behälter (3) im Füllzustand (23) fluidisch miteinander verbunden sind, sodass im Behälter (3) bevorratete Flüssigkeit in die Aufnahmestruktur (7) gelangt,
- wobei die Aufnahmestruktur (7) und der Behälter (3) im Verdampfungszustand (24) fluidisch voneinander getrennt sind, sodass ein Strömen von Flüssigkeit vom Behälter (3) in die Aufnahmestruktur (7) unterbunden ist,
wobei die Aufnahmestruktur (7) ein vorgegebenes Gesamtvolumen zum Aufnehmen der Flüssigkeit aufweist,
- wobei der Inhalator (1) eine Steuereinrichtung (21) aufweist, die derart ausgestaltet ist, dass sie den Verdampfer (4) im Verdampfungszustand (24) elektrisch versorgt, sodass die in der Aufnahmestruktur (7) aufgenommene Flüssigkeit verdampft,
**dadurch gekennzeichnet,**
**dass** die Verdampfereinrichtung (2) und der Behälter (3) zum Verstellen zwischen dem Füllzustand (23) und dem Verdampfungszustand (24) relativ zueinander beweglich sind.

2. Inhalator nach Anspruch 1,
**dadurch gekennzeichnet,**
**dass** die Steuereinrichtung (21) derart ausgestaltet ist, dass sie die elektrische Versorgung des Verdampfers (4) im Füllzustand (23) unterbricht.

3. Inhalator nach Anspruch 1 oder 2,
**dadurch gekennzeichnet,**
**dass** die Steuereinrichtung (21), derart ausgestaltet ist, dass sie ein Verstellen vom Verdampfungszustand (24) in den Füllzustand (23) verhindert, bis die in der Aufnahmestruktur (7) aufgenommene Flüssigkeit vollständig verdampft ist.

4. Inhalator nach einem der Ansprüche 1 bis 3,
**dadurch gekennzeichnet,**
**dass** der Inhalator (1) derart ausgestaltet ist, dass im Füllzustand (23) ein vollständiges Befüllen der Aufnahmestruktur (7) mit der im Behälter (3) bevorraten Flüssigkeit erfolgt.

5. Inhalator nach einem der Ansprüche 1 bis 4,
**dadurch gekennzeichnet,**
**dass** der Verdampfer (4) die Aufnahmestruktur (7) aufweist.

6. Inhalator nach Anspruch 5,
**dadurch gekennzeichnet,**
**dass** der Verdampfer (4) als eine elektrisch leitende Verdampferkeramik (6) ausgebildet ist.

7. Inhalator nach Anspruch 6,
**dadurch gekennzeichnet,**
- **dass** die Verdampferkeramik (6) derart ausgestaltet ist, dass sie im Betrieb bei elektrischer Versorgung zum Verdampfen der in der Aufnahmestruktur (7) aufgenommenen Flüssigkeit homogen Wärme in einem thermischen Betriebsbereich zwischen einer Betriebsanfangstemperatur und einer Betriebsendtemperatur erzeugt,
- **dass** der Inhalator (1), insbesondere die Verdampfereinrichtung (2), zwei elektrische Anschlüsse (5) zur elektrischen Versorgung der Verdampferkeramik (6) aufweist, wobei ein elektrischer Strompfad (8) zur elektrischen Versorgung der Verdampferkeramik (6) durch die Anschlüsse (5) und durch die Verdampferkeramik (6) verläuft,
- **dass** der Inhalator (1) zumindest einen im Strompfad (8) angeordneten Sperrleiter (9) aufweist, welcher wärmeübertragend mit der Verdampferkeramik (6) verbunden ist,
- **dass** der zumindest eine Sperrleiter (9) derart ausgestaltet ist, dass er beim Überschreiten der Betriebsendtemperatur einen sprunghaft ansteigenden elektrischen Widerstand aufweist.

8. Inhalator nacheinem der Ansprüche 1 bis 7,
**dadurch gekennzeichnet,**
- **dass** der Behälter (3) einen Behälterauslass (30) aufweist,
- **dass** die Verdampfereinrichtung (2) eine an einer Aufnahmefläche (31) der Aufnahmestruktur (7) anschließende Dichtung (33) aufweist,
- **dass** die Aufnahmefläche (31) im Füllzustand (23) an den Behälterauslass (30) angrenzt und die Aufnahmestruktur (7) somit fluidisch mit dem Behälter (3) verbunden ist,
- **dass** die Dichtung (33) den Behälterauslass (30) im Verdampfungszustand (24) abdichtet.

9. Inhalator nach Anspruch 8,
**dadurch gekennzeichnet,**
- **dass** die Aufnahmestruktur (7) eine zur Aufnahmefläche (31) beabstandete Ausgabefläche (32) aufweist,
- **dass** im Verdampfungszustand (24) und bei elektrische Versorgung des Verdampfers (4) die in der Aufnahmestruktur (7) verdampfte Flüssigkeit aus der Ausgabefläche (32) austritt.

10. Inhalator nach Anspruch 8 oder 9,
**dadurch gekennzeichnet,**
- **dass** der Behälter (3) eine Innenkontur (29) aufweist, welche den Behälterauslass (30) aufweist,
- **dass** die Verdampfungseinrichtung (2) zum Verstellen zwischen dem Füllzustand (23) und dem Verdampfungszustand (24) entlang der Innenkontur (29) geführt ist.

11. Inhalator nach Anspruch 10,
**dadurch gekennzeichnet,**
**dass** der Behälter (3) in der Art eines Zylinders ausgebildet ist, in welcher die Verdampfereinrichtung (3) in der Art eines Kolbens geführt ist.

12. Inhalator nach Anspruch 10 oder 11,
**dadurch gekennzeichnet,**
**dass** der Behälter (3) einen Kamin (34) für die mit dem Verdampfer (4) verdampfte Flüssigkeit bildet.

13. Inhalator nach einem der Ansprüche 1 bis 12,
**dadurch gekennzeichnet,**
**dass** die Aufnahmestruktur (7) Poren aufweist, insbesondere als eine Porenstruktur ausgebildet ist.

## Claims

1. Inhaler (1) for inhaling an aerosol,
- having a container (3) for storing a liquid,
- having an evaporator apparatus (2) which has an electric evaporator (4) for evaporating the liquid and thus producing the aerosol,
- wherein the evaporator apparatus (2) has a receiving structure (7) for receiving the liquid,
- wherein the inhaler (1) is adjustable between a filling state (23) and an evaporation state (24),
- wherein the receiving structure (7) and the container (3), in the filling state (23), are fluidically connected to one another, so that liquid stored in the container (3) enters the receiving structure (7),
- wherein the receiving structure (7) and the container (3), in the evaporation state (24), are fluidically separated from one another, so that a flowing of liquid from the container (3) into the receiving structure (7) is prevented,
wherein the receiving structure (7) has a predetermined total volume for receiving the liquid,
- wherein the inhaler (1) has a control apparatus (21) which is configured in such a manner that it electrically supplies the evaporator (4) in the evaporation state (24), so that the liquid received in the receiving structure (7) evaporates,
**characterized in that**
the evaporator apparatus (2) and the container (3), for adjusting between the filling state (23) and the evaporation state (24), are movable relative to one another.

2. Inhaler according to claim 1,
**characterized in that**
the control apparatus (21) is configured in such a manner that it interrupts the electrical supply to the evaporator (4) in the filling state (23).

3. Inhaler according to claim 1 or 2,
**characterized in that**
the control apparatus (21) is configured in such a manner that it prevents an adjusting from the evaporation state (24) into the filling state (23) until the liquid received in the receiving structure (7) is completely evaporated.

4. Inhaler according to any one of claims 1 to 3,
**characterized in that**
the inhaler (1) is configured in such a manner that in the filling state (23) a complete filling of the receiving structure (7) with the liquid stored in the container (3) takes place.

5. Inhaler according to any one of claims 1 to 4,
**characterized in that**
the evaporator (4) has the receiving structure (7).

6. Inhaler according to claim 5,
**characterized in that**
the evaporator (4) is designed as an electrically conductive evaporator ceramic (6).

7. Inhaler according to claim 6,
**characterized in that**
- the evaporator ceramic (6) is configured in such a manner that during operation and upon electrical supply it homogeneously generates heat in a thermal operating range between an operation starting temperature and an operation end temperature in order to evaporate the liquid received in the receiving structure (7),
- the inhaler (1), in particular the evaporator apparatus (2), has two electrical connections (5) for the electrical supply to the evaporator ceramic (6), wherein an electrical current path (8) for the electrical supply to the evaporator ceramic (6) runs through the connections (5) and through the evaporator ceramic (6),
- the inhaler (1) has at least one blocking conductor (9) which is arranged in the current path (8) and which is heat-transmittingly connected to the evaporator ceramic (6),
- the at least one blocking conductor (9) is configured in such a manner that it has an abruptly increasing electrical resistance when the operation end temperature is exceeded.

8. Inhaler according to any one of claims 1 to 7,
**characterized in that**
- the container (3) has a container outlet (30),
- the evaporator apparatus (2) has a seal (33) adjoining a receiving surface (31) of the receiving structure (7),
- the receiving surface (31), in the filling state (23), adjoins the container outlet (30) and the receiving structure (7) is thus fluidically connected to the container (3),
- the seal (33) seals the container outlet (30) in the evaporation state (24).

9. Inhaler according to claim 8,
**characterized in that**
- the receiving structure (7) has a dispensing surface (32) spaced apart from the receiving surface (31),
- in the evaporation state (24) and when the evaporator (4) is electrically supplied, the liquid evaporated in the receiving structure (7) exits from the dispensing surface (32).

10. Inhaler according to claim 8 or 9,
**characterized in that**
- the container (3) has an inner contour (29) which has the container outlet (30),
- the evaporation apparatus (2), for adjusting between the filling state (23) and the evaporation state (24), is guided along the inner contour (29).

11. Inhaler according to claim 10,
**characterized in that**
the container (3) is formed in the manner of a cylinder in which the evaporator apparatus (3) is guided in the manner of a piston.

12. Inhaler according to claim 10 or 11,
**characterized in that**
the container (3) forms a chimney (34) for the liquid evaporated with the evaporator (4).

13. Inhaler according to any one of claims 1 to 12,
**characterized in that**
the receiving structure (7) has pores, in particular is formed as a pore structure.

## Revendications

1. Inhalateur (1) pour l'inhalation d'un aérosol,
- avec un récipient (3) pour le stockage d'un liquide,
- avec un dispositif évaporateur (2) qui présente un évaporateur électrique (4) pour l'évaporation du liquide et ainsi la génération de l'aérosol,
- dans lequel le dispositif évaporateur (2) présente une structure de réception (7) pour la réception du liquide,
- dans lequel l'inhalateur (1) est réglable entre un état de remplissage (23) et un état d'évaporation (24),
- dans lequel la structure de réception (7) et le récipient (3) sont reliés de manière fluidique l'un à l'autre dans l'état de remplissage (23) de sorte que du liquide stocké dans le récipient (3) parvienne dans la structure de réception (7),
- dans lequel la structure de réception (7) et le récipient (3) sont séparés de manière fluidique l'un de l'autre dans l'état d'évaporation (24) de sorte qu'un écoulement de liquide du récipient (3) dans la structure de réception (7) soit évité,
dans lequel la structure de réception (7) présente un volume global prédéfini pour la réception du liquide,
- dans lequel l'inhalateur (1) présente un dispositif de commande (21) qui est configuré de manière à alimenter électriquement l'évaporateur (4) dans l'état d'évaporation (24) de sorte que le liquide reçu dans la structure de réception (7) s'évapore,
**caractérisé en ce que**
le dispositif évaporateur (2) et le récipient (3) sont mobiles l'un par rapport à l'autre pour le réglage entre l'état de remplissage (23) et l'état d'évaporation (24).

2. Inhalateur selon la revendication 1,
**caractérisé en ce que**
le dispositif de commande (21) est configuré de manière à interrompre l'alimentation électrique de l'évaporateur (4) dans l'état de remplissage (23).

3. Inhalateur selon la revendication 1 ou 2,
**caractérisé en ce que**
le dispositif de commande (21) est configuré de manière à empêcher un réglage de l'état d'évaporation (24) dans l'état de remplissage (23) jusqu'à ce que le liquide reçu dans la structure de réception (7) soit complètement évaporé.

4. Inhalateur selon l'une quelconque des revendications 1 à 3,
**caractérisé en ce que**
l'inhalateur (1) est configuré de telle manière que dans l'état de remplissage (23), un remplissage complet de la structure de réception (7) avec le liquide stocké dans le récipient (3) soit effectué.

5. Inhalateur selon l'une quelconque des revendications 1 à 4,
**caractérisé en ce que**
l'évaporateur (4) présente la structure de réception (7).

6. Inhalateur selon la revendication 5,
**caractérisé en ce que**
l'évaporateur (4) est formé comme une céramique d'évaporateur (6) électroconductrice.

7. Inhalateur selon la revendication 6,
**caractérisé en ce que**
- la céramique d'évaporateur (6) est configurée de manière à générer en fonctionnement lors de l'alimentation électrique pour l'évaporation du liquide reçu dans la structure de réception (7) de la chaleur de manière homogène dans une plage de fonctionnement thermique entre une température de début de fonctionnement et une température de fin de fonctionnement,
- l'inhalateur (1), en particulier le dispositif évaporateur (2) présente deux raccords électriques (5) pour l'alimentation électrique de la céramique d'évaporateur (6), dans lequel un trajet de courant (8) électrique pour l'alimentation électrique de la céramique d'évaporateur (6) s'étend à travers les raccords (5) et la céramique d'évaporateur (6),
- l'inhalateur (1) présente au moins un conducteur de blocage (9) agencé dans le trajet de courant (8), conducteur qui est relié de manière à transmettre la chaleur à la céramique d'évaporateur (6),
- le au moins un conducteur de blocage (9) est configuré de manière à présenter lors du dépassement de la température de fin de fonctionnement une résistance électrique qui augmente brusquement.

8. Inhalateur selon l'une quelconque des revendications 1 à 7,
**caractérisé en ce que**
- le récipient (3) présente une sortie de récipient (30),
- le dispositif évaporateur (2) présente un joint d'étanchéité (33) contigu à une surface de réception (31) de la structure de réception (7),
- la surface de réception (31) dans l'état de remplissage (23) jouxte la sortie de récipient (30) et la structure de réception (7) est ainsi reliée de manière fluidique au récipient (3),
- le joint d'étanchéité (33) rend étanche la sortie de récipient (30) dans l'état d'évaporation (24).

9. Inhalateur selon la revendication 8,
**caractérisé en ce que**
- la structure de réception (7) présente une surface de sortie (32) espacée de la surface de réception (31),
- dans l'état d'évaporation (24) et lors de l'alimentation électrique de l'évaporateur (4), le liquide évaporé dans la structure de réception (7) sort de la surface de sortie (32).

10. Inhalateur selon la revendication 8 ou 9,
**caractérisé en ce que**
- le récipient (3) présente un contour intérieur (29) qui présente la sortie de récipient (30),
- le dispositif évaporateur (2) est guidé pour le réglage entre l'état de remplissage (23) et l'état d'évaporation (24) le long du contour intérieur (29).

11. Inhalateur selon la revendication 10,
**caractérisé en ce que**
le récipient (3) est formé comme un cylindre, dans lequel le dispositif évaporateur (3) est guidé comme un piston.

12. Inhalateur selon la revendication 10 ou 11,
**caractérisé en ce que**
le récipient (3) forme une cheminée (34) pour le liquide évaporé avec l'évaporateur (4).

13. Inhalateur selon l'une quelconque des revendications 1 à 12,
**caractérisé en ce que**
la structure de réception (7) présente des pores, en particulier est formée comme une structure à pores.
